# EUROPÄISCHE PATENTANMELDUNG

(11) **EP 4 111 904 A1**
(43) Veröffentlichungstag der Anmeldung: **04.01.2023**
(21) Anmeldenummer: 22182045.9
(22) Anmeldetag: 29.06.2022
(51) Int. Cl.: A44C 5/14, G04B 37/16, G06F 1/16, A61B 5/00

(54) **GEHÄUSE MIT EINEM ARMBAND ZUR AUFNAHME EINER ELEKTRONISCHEN UND/ODER MECHANISCHEN EINRICHTUNG.**

(30) Priorität: 30.06.2021 DE 102021116801
(71) Anmelder: ILPER Elektronik GmbH & Co. KG, 27412 Tarmstedt (DE)
(72) Erfinder: Ilper, Bernd, 27412 Tarmstedt (DE)
(74) Vertreter: Rauch, Udo

(57) **Zusammenfassung**

Ein Gehäuse (1) besteht aus einem Unterteil (2) und einem Deckel (3). Das Unterteil (2) weist einen Boden (4) und einen hochgezogenen Rand (5) auf. Im Rand (5) befinden sich gegenüberliegend zwei Schlitze (6, 7), in denen die Enden (31, 32) eines einteiligen Armbandes (30) eingeführt und innerhalb des Gehäuses (1) über Dorne (9, 10) gelegt werden.

Wenn der Deckel (3) geschlossen wird, legt sich seine Unterseite auf oder über die Dorne (9, 10), so dass die Enden des Armbandes (30) nicht mehr von den Dornen (9, 10) gezogen werden können.

Der Deckel (3) wird am Unterteil (2) mittels einer nur mit einem Werkzeug zu öffnenden Schnappverbindung gehalten.

Das Gehäuse (1) eignet sich zur Aufnahme eines Transponders, der von dementen Personen in einer Pflegeeinrichtung getragen werden sollte, damit rechtzeitig bemerkt werden kann, wenn diese Personen das Gebäude oder das Gelände der Pflegeeinrichtung verlassen.

## Beschreibung

Die Erfindung bezieht sich auf ein zur Aufnahme einer elektronischen und/oder mechanischen Einrichtung vorgesehenes Gehäuse mit einem Armband zum Befestigen des Gehäuses, insbesondere am Handgelenk einer Person, und mit einer Einrichtung, die ein Lösen des Armbandes zum Entfernen des Gehäuses vom Handgelenk mittels eines Werkzeuges erlaubt.

Ein solches Gehäuse mit Armband wird zum Beispiel benötigt, um einen Transponder aufzunehmen, mit dem die Bewegung von Personen nachvollzogen werden kann, die das Gehäuse mit Transponder am Armgelenk tragen. In Pflegeeinrichtungen, wo Personen untergebracht sind, die sich nicht mehr orientieren können, erhalten diese einen Transponder. So kann bemerkt werden, wenn eine einen Transponder tragende Person einen auf den Transponder ansprechenden Sensor am Ausgang der Pflegeeinrichtung passiert. Da den betroffenen Personen der Zweck des Transponders aber nicht bekannt ist, versuchen sie häufig das Gehäuse zu entfernen, indem sie das Armband lösen.

Es sind schon verschiedene Vorschläge bereitet worden, um dies zu verhindern. So wird z. B. in der DE 10 2005 016 425 B4 vorgeschlagen, das Armband mit einem Verschluss zu versehen, der magnetisch geöffnet werden kann. Nur das Pflegepersonal besitzt hierfür einen Magnetschlüssel. Es ist aber gerade der Verschluss, der von den betroffenen Personen häufig als störend empfunden wird, weil er gegen das Handgelenk drückt. Bei dem vergeblichen Versuch, das Gehäuse mit Armband vom Handgelenk zu entfernen, kann es zu Verletzungen kommen.

Die Erfindung geht von der Aufgabe aus, eine Gehäuse/Armband-Kombination zu entwickeln, die ebenfalls nur durch ein Löse-Werkzeug gelöst werden kann, aber dennoch angenehm zum Beispiel am Handgelenk getragen werden kann, so dass nicht das Bedürfnis entsteht, sie zu entfernen.

Zur Lösung der Aufgabe sieht die Erfindung vor, dass das Gehäuse ein Unterteil und einen über dem Unterteil liegenden Deckel aufweist, dass das Armband einteilig ist und zwei Enden aufweist, wobei mindestens eines der Enden auf der zum Deckel gerichteten Innenseite des Unterteils innerhalb der Erstreckungsabmessung des Deckels, also beispielsweise innerhalb des Durchmessers des Deckels, lösbar befestigt ist, und dass der Deckel lösbar mit dem Unterteil verbunden ist, wobei die Verbindung des Deckels mit dem Unterteil so eingerichtet ist, dass sie nur mit dem Löse-Werkzeug lösbar ist.

Der Kern der Erfindung liegt somit darin, dass das Gehäuse zwei Aufgaben übernimmt. Zum einem kann es eine elektronische oder mechanische Einrichtung (z. B. einen Transponder) aufnehmen und zum anderen dient es als Verschluss. Es wird somit kein gesonderter Verschluss am Armband vorgesehen, der als störend empfunden wird.

Zur Realisierung der Verschlussfunktion befindet sich eine lösbare Verbindung des einen Endes des Armbandes innerhalb des Gehäuses vor, die nur erreicht werden kann, wenn der Deckel vom Unterteil entfernt wird. Das andere Ende des Armbandes kann in gleicher Weise lösbar innerhalb des Gehäuses befestigt werden. Es kann aber auch eine feste Verbindung mit dem Gehäuse realisiert werden, da es zum Lösen des Armbandes ausreicht, eines seiner Enden vom Gehäuse zu lösen. Für eine feste Verbindung kann das andere Ende des Armbandes einstückig mit dem Unterteil ausgeführt sein oder mit diesem in anderer Weise nicht lösbar verbunden werden.

Damit das Gehäuse trotz eines geschlossenen Armbandes nicht beispielsweise von einem Hand- oder Fußgelenk entfernt werden kann, ist das Armband undehnbar ausgeführt, es kann also nicht übermäßig gedehnt werden. Der Begriff 'undehnbar' beinhaltet eine geringe Dehnbarkeit, die jedenfalls nicht so weit geht, dass das geschlossene Armband über die Hand gezogen werden könnte. Je weniger dehnbar das Armband ist, desto eher kann es locker am Hand- oder Fußgelenk getragen werden, ohne dass die Gefahr besteht, dass es über das Gelenk gezogen wird. Beispielsweise kann das Armband so hergerichtet sein, dass es sich unter Aufbringung einer von einem Menschen aufbringbaren Kraft nicht dehnen lässt. Eine von einem Menschen aufbringbare Kraft beträgt beispielsweise 1300 N Zugkraft oder weniger. Ferner kann das Armband so hergerichtet sein, dass es eine Gleichmaßdehnung A_{g} aufweist, so dass unter Aufbringung einer Zugkraft bzw. einer Dehnung das Ausgangsmaß bzw. Anfangsmesslänge Lo sich nicht oder unwesentlich ändert. Dabei gilt insbesondere A_{g}=Lₚₘ/L₀-1, mit Lₚₘ als plastischer Längenänderung und Lo als Anfangsmesslänge, und wobei gilt A_{g} = 0 oder 0 ≤ A_{g} ≤ 0,05 oder ≤ 0,01.

Vorzugsweise sind beide Enden des Armbandes auf der zum Deckel gerichteten Innenseite des Unterteils innerhalb der Erstreckungsabmessung, also beispielsweise des Durchmessers, des Deckels angeordnet bzw. lösbar befestigt. Insbesondere, wenn die Verbindungen gleichartig aufgebaut sind, wird die Konstruktion des Gehäuses vereinfacht.

Eine einfache Art der Befestigung besteht darin, dass sich auf der zum Deckel gerichteten Innenseite des Unterteils mindestens ein Dorn befindet und dass das mindestens eine Ende des Armbandes ein oder mehrere zum Dorn passende Löcher aufweist. So können entweder beide Enden des Armbandes mit dem einen Dorn befestigt werden oder aber jedes der zwei Enden des Armbandes mit einem eigenen Dorn innerhalb des Gehäuses befestigt werden.

Die Löcher werden, wie bei einem Gürtel, in Längsrichtung des Armbandes angeordnet. Damit kann ein beliebiger Durchmesser des geschlossenen Armbandes eingestellt werden. Sollten beide Enden des Armbandes im Gehäuse befestigt werden, so können auch zwei Dorne, ein Dorn für jedes Ende, vorgesehen werden. Die Länge des Armbandes kann dann durch korrekt positionierte Einbringung der Löcher in das Armband eingestellt werden.

Um einen sicheren Halt des Armbandes zu gewährleisten, können für jedes Ende des Armbandes auch zwei oder mehrere Dorne vorgesehen werden.

Wenn je ein Dorn vorgesehen ist, brauchen sich die Enden des Armbandes im Gehäuse nicht zu überlappen, so dass ausreichend Platz zur Unterbringung eines Transponders verbleibt. Im Prinzip können aber auch beide Enden auf einen Dorn aufgesteckt werden.

Damit die Enden des Armbandes nicht von den Dornen rutschen können, ist vorgesehen, dass jeder Dorn bis zur Unterseite des Deckels reicht, wenn dieser am Unterteil befestigt ist. Somit kann das Armband bei einem geschlossenen Deckel nicht vom Dorn bzw. den Dornen gezogen werden.

Um die Enden des Armbandes noch besser zu sichern, ist in der Unterseite des Deckels je eine Mulde für jeden Dorn ausgebildet, in die dieser bei einem geschlossenen Deckel hineinragt.

Um das mindestens eine Ende des Armbandes in das Gehäuse einführen zu können, ist vorgesehen, dass das Unterteil einen Boden mit einem zum Deckel hin hochgezogenen Rand aufweist, wobei sich im Rand mindestens ein Schlitz befindet, durch den das mindestens eine Ende des Armbandes eingeführt ist, dass der Dorn vor dem Schlitz liegt und dass der Deckel am Rand lösbar befestigt ist.

Zum Schließen des Armbandes wird bei einem offenen Deckel das mindestens eine Ende des Armbandes durch den Schlitz in das Gehäuse geführt und auf den Dorn aufgesteckt. Dann wird der Deckel verschlossen, so dass das Armband nicht mehr zurückgezogen werden kann. Das Armband kann erst dann gelöst werden, wenn der Deckel entfernt und das Ende des Armbandes vom Dorn genommen wird.

Der Deckel ist vorzugsweise mittels der gesicherten Löse-Einrichtung mit dem Unterteil lösbar verbunden. Die gesicherte Löse-Einrichtung kann als eine Schnappverbindung ausgeführt sein, mittels welcher der Deckel mit dem Unterteil verbindbar ist. Die Schnappverbindung kann mindestens zwei voneinander beabstandete Federhaken aufweisen, deren Enden bei einem mit dem Unterteil verbundenen Deckel freiliegen, so dass das Werkzeug an den freiliegenden Enden ansetzbar ist.

Zum Lösen des Deckels müssten daher mindestens zwei Federhaken gleichzeitig zurückgezogen werden, was insbesondere, wenn sie etwas vertieft im Unterteil liegen, für eine einzelne Person ohne Löse-Werkzeug nicht oder nur äußerst schwierig möglich ist. Mithin ist die gesicherte Löse-Einrichtung derart realisiert, dass sie nur dann die Verbindung des Deckels mit dem Unterteil freigibt, wenn das korrekte Löse-Werkzeug angelegt und korrekt bedient wird.

Ein geeignetes Löse-Werkzeug zum Bedienen der gesicherten Löse-Einrichtung besteht aus einer Gabel mit mindestens zwei Zinken, deren Enden paarweise den gleichen Abstand wie jeweils zwei Federhaken des Gehäuses haben.

Zur Sicherung der Löse-Einrichtung kann an Deckel oder Unterteil, beispielsweise zwischen den beiden Federhaken, weiter beispielsweise an dem Unterteil, eine Sicherungsnase angeordnet sein, die das Löse-Werkzeug in der Einsatzposition sichert und/oder als Widerlager für das Löse-Werkzeug bei der Betätigung der Löse-Einrichtung eingerichtet ist. Mit anderen Worten kann die Sicherungsnase so hergerichtet sein, dass das Löse-Werkzeug über die Sicherungsnase in Richtung des Unterteils bzw. in Richtung der Federhaken überschnappt und in einer Einsatzposition einhakt bzw. einrastet. Hierzu kann das Löse-Werkzeug einen Sicherungsbügel aufweisen zum Einhaken unter der Sicherungsnase der Löse-Einrichtung. In der Einsatzposition des Löse-Werkzeugs kann die Löse-Einrichtung betätigt werden, so dass die Verbindung zwischen Deckel und Unterteil freigegeben wird. Hierbei ist besonders vorteilhaft, wenn die Löse-Einrichtung mehrere, also zumindest zwei, Verbindungen wie die genannten Federhaken aufweist, die gleichzeitig betätigt werden müssen, um das Gehäuse zu öffnen. Die Sicherungsnase und/oder die mehreren Verbindungen der Löse-Einrichtung verhindern oder erschweren somit eine manuelle Betätigung der Löse-Einrichtung ohne das komplementäre Löse-Werkzeug, so dass ein Öffnen des Gehäuses ohne das komplementäre Löse-Werkzeug in vorteilhafter Weise verunmöglicht ist.

Beispielsweise kann die Sicherungsnase weiter so hergerichtet sein, dass das Löse-Werkzeug in der Einsatzposition an der Sicherungsnase verrastet oder verhakt, so dass es auch bei schwierigen Sichtbedingungen oder im Falle eines unkooperativen Anwenders des Gehäuses möglich ist, die sichere Einnahme der Einsatzposition des Löse-Werkzeugs am Gehäuse festzustellen, wenn nämlich das Löse-Werkzeug beispielsweise nurmehr in einem bestimmten Lösewinkel oder mittels eines Löseschwenks aus der Einsatzposition entnehmbar ist, ansonsten aber in der Einsatzposition sicher verrastet bzw. verhakt. Dann kann die Löse-Einrichtung zuverlässig auch in schwierigen Bedingungen bedient werden, ohne dass das Löse-Werkzeug von den Verbindern wie den Federhaken abrutscht.

Die Erfindung bezieht sich weiterhin auf das Gehäuse selbst, wie es im Anspruch 10 definiert ist, und dem Löse-Werkzeug zum Bedienen der Löse-Vorrichtung des Gehäuses.

Im Folgenden soll anhand eines Ausführungsbeispiels die Erfindung näher erläutert werden. Dazu zeigen jeweils in einer perspektivischen Darstellung:
- Fig. 1: ein geschlossenes Gehäuse,
- Fig. 2: das halb geöffnete Gehäuse,
- Fig. 3: das offene Gehäuse,
- Fig. 4: das offene Gehäuse mit einem eingelegten Armband,
- Fig. 5: in der Draufsicht ein Werkzeug und
- Fig. 6: in einer perspektivischen Darstellung ein geschlossenes Gehäuse gemäß Fig. 1, an dem das Werkzeug gemäß Fig. 5 zum Öffnen des Gehäuses angesetzt ist.

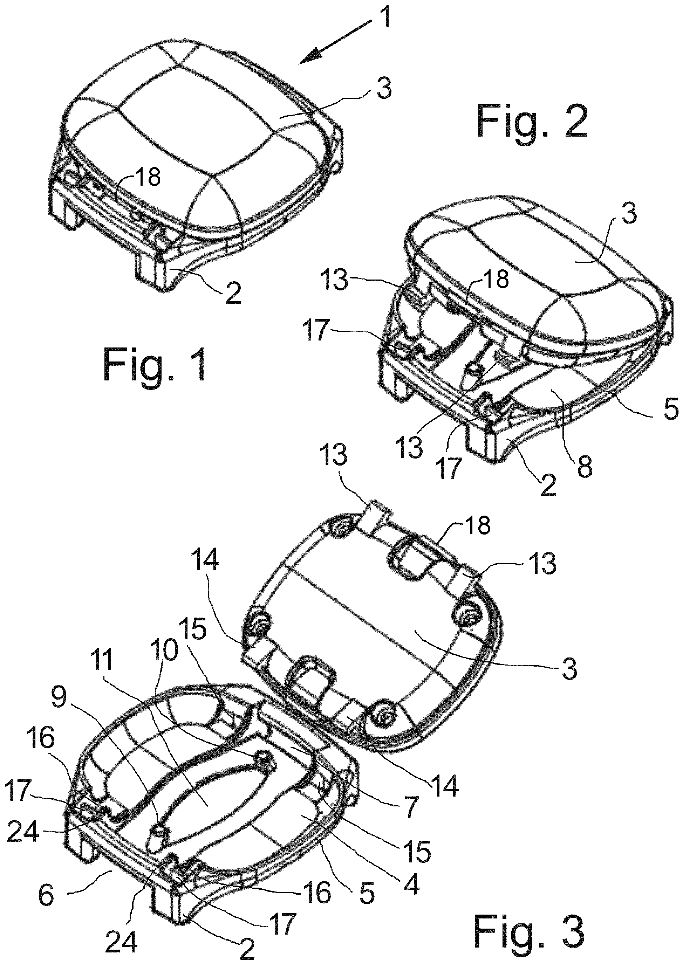

Zunächst wird auf die Figuren 1, 2 und 3 Bezug genommen. Ein Gehäuse 1 gemäß Fig. 1 besteht aus einem Unterteil 2 und einem Deckel 3, wobei das Unterteil 2 aus einem Boden 4 und einem hochgezogenen Rand 5 besteht. In zwei gegenüberliegenden Randabschnitten des Unterteils 2 ist je ein Schlitz 6, 7 ausgebildet. Zwischen den Schlitzen verläuft im Boden 4 eine flache längliche Vertiefung 8, wobei sich in der Vertiefung 8 hinter jedem Schlitz 6, 7 jeweils ein senkrecht oder schräg zum Boden ausgerichteter Dorn 9, 10 befindet. Zwischen den Dornen 9, 10 ist eine Öffnung 11 im Boden 4 vorgesehen. Bei einer schrägen Ausrichtung sind die Dorne 9, 10 leicht nach innen geneigt.

Der Deckel 3 ist mit seiner Außenkontur, wie insbesondere Fig. 1 zeigt, auf den Rand 5 aufgesetzt, wobei über den gesamten Umfang zwischen dem Rand 5 und dem Deckel 3 ein geschlossener Kontakt vorliegt. Dadurch ist der Boden 4 vollständig vom aufgesetzten Deckel 3 überdeckt.

Ein hier nicht dargestellter Transponder kann zwischen dem Boden 4 und dem Deckel 3 untergebracht werden. Denkbar wäre es auch, den Transponder im Deckel 3 anzuordnen, wobei die zum Unterteil 2 weisende Unterseite des Deckels 3 flach auf dem Boden 4 zu liegen kommt, wodurch zwischen der Unterseite und der Sohle der Vertiefung 8 ein Kanal entsteht.

An zwei gegenüberliegenden Seiten des Deckels 3 befindet sich je ein aus zwei Nasen 13 bzw. 14 bestehendes Nasenpaar, das dem Verschluss des Deckels 3 am Unterteil 2 dient. Dazu befinden sich in gegenüberliegenden Abschnitten des Randes 5 des Unterteils 2 jeweils paarweise zwei Aussparungen 15 bzw. 16, wobei die Aussparungen 15 des einen Paares einen Hinterschnitt besitzen, die die dort eingesetzten Nasen 14 untergreifen. Die gegenüberliegenden Aussparungen 16 sind mit Federhaken 17 versehen, deren Rasten die eingesetzten Nasen 13 übergreifen und sichern. Zum Lösen des Deckels 3 vom Unterteil 2 werden die beiden Federhaken 17 gleichzeitig radial nach außen gedrückt, so dass die Rasten den Weg für die Nasen 13 freigeben. Dadurch kann der Deckel 3 aufgeklappt werden, wobei die gegenüberliegenden Nasen 14 aus den Aussparungen 15 mit den festen Hinterschnitten herausgedreht werden.

Zum Schließen des Deckels 3 werden die Nasen 14 des einen Nasenpaares in die Aussparungen 15 mit den Hinterschnitten gesetzt. Anschließend wird der Deckel 3 heruntergeklappt, wobei sich die Nasen 14 in den Aussparungen 15 mit den Hinterschnitten gelenkig gedreht werden, bis die Nasen 13 des anderen Nasenpaares die Federhaken 17 erreichen. Wenn der Deckel 3 weiter nach unten gedrückt wird, werden diese Nasen 13 die Federhaken 17 federnd nach außen drücken, bis die Rasten der Federhaken 17 schließlich über die Nasen 13 zurückschnappen.

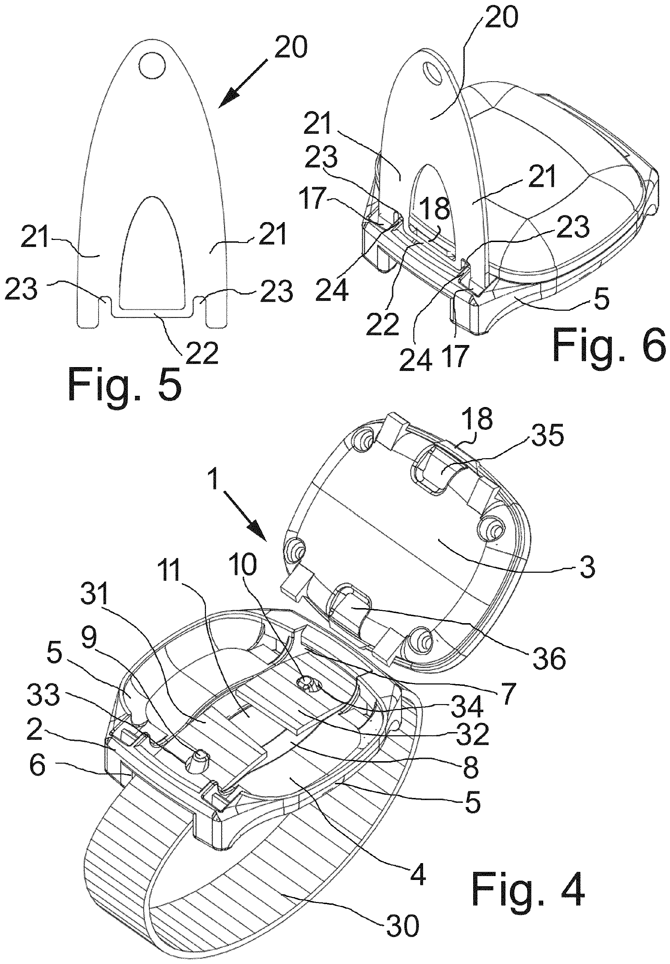

Zum Öffnen des Deckels 2 müssen beide Federhaken 17 gleichzeitig zurückgezogen werden. Dazu ist ein Löse-Werkzeug 20 vorgesehen, das in Fig. 5 dargestellt ist. Es hat die Form einer zweizinkigen Gabel, wobei der Abstand der Enden der beiden Zinken 21 dem Abstand der beiden Federhaken 17 entspricht. Eine Querstrebe bzw. Sicherungsbügel 22 verläuft zwischen den beiden Zinken 21, um diese einerseits zu stabilisieren, andererseits aber kann mit dem Sicherungsbügel 22 die Sicherungsnase 18 hintergriffen werden. Zu beiden Seiten der Querstrebe 22, befindet sich je ein Einschnitt 23.

Mit anderen Worten taucht gemäß Fig. 6 die Querstrebe 22 beim Ansetzen des Werkzeuges 20 an das Gehäuse 1 in einen von zwei Querstegen 24 begrenzten und gegenüber der Sicherungsnase 18 vertieften Bereich im Rand 5 ein, wodurch die Querstege 24 in die Einschnitte 23 eintauchen. Dadurch ist eine sichere Führung des Werkzeuges 20 gegeben, wenn dieses verschwenkt wird, damit die Enden der Zinken 21 die Federhaken 17 nach außen drücken können. Der Sicherungsbügel 22 weist also in besonders bevorzugter und synergistischer Ausgestaltung drei Funktionen auf: Erstens stabilisiert er die beiden Zinken 21, so dass diese gleichmäßig und/oder gleichzeitig verschwenken; zweitens verkantet oder verrastet das Löse-Werkzeug 20 mittels des Sicherungsbügels 22 an der Sicherungsnase 18 bei der Betätigung; und drittens ist der Sicherungsbügel 22 das Widerlager zum Gegenhalten der Andrückkraft des Löse-Werkzeugs an der Löse-Einrichtung bzw. an den Federhaken 17 der Löse-Einrichtung. Durch das Gegenhalten (Widerlager) verstärkt sich zugleich auch die Verkantung bzw. Verrastung an der Sicherungsnase 18, da das Löse-Werkzeug 20 dabei unter der Sicherungsnase 18 angedrückt wird und bei Aufbringung einer Zugkraft zum Lösen des Werkzeugs aus der Einsatzposition die Verkantung bzw. Verrastung erst überwunden werden müsste.

In der Fig. 4 ist das offene Gehäuse mit einem eingelegten Armband 30 dargestellt. Das Armband ist einteilig und weist zwei Enden 31, 32 auf, die hier aus Darstellungsgründen nur mit je einem Loch 33, 34 versehen sind. Das Armband ist biegeelastisch, kann also gebogen werden, ist aber nicht bzw. kaum dehnbar, so dass es nicht verlängert werden kann.

Um das Gehäuse 1 mit einem Löcher 33, 34 aufweisenden Armband 30 zu versehen, werden die beiden Enden 31, 32 des Armbandes 30 durch die Schlitze 6, 7 in das Unterteil 2 eingeführt und die Dorne 9, 10 in die Löcher 33, 34 gesteckt. Die Enden des Armbandes 30 liegen dann in der Vertiefung 8, wobei die Unterseite des Deckels 3 auf den Enden aufliegt und sie in der Vertiefung 8 hält.

Wenn der Deckel 3 geschlossen ist, wie in Fig 1. (ohne Armband), reichen die Enden der Dorne 9, 10 bis zur Unterseite des Deckels, so dass die Enden nicht von ihnen abrutschen können. Dazu befinden sich in der Unterseite des Deckels zwei Mulden 35, 36, in die die Dorne 9, 10 bei geschlossenem Deckel 3 eintauchen. Auch ohne einen direkten Kontakt der Dorne 9, 10 mit der Unterseite des Deckels 3 würde dies schon ausreichen, ein Abrutschen des Armbandes 30 zu verhindern. Vorzugsweise befinden sich die Mantelflächen der Dorne 9, 10 sehr nahe der zur Mitte des Deckels liegenden Wand der jeweiligen Mulde 35, 36 oder kontaktieren sogar diese Wand.

Bei einer solchen Ausbildung des Gehäuses 1 können somit die Enden 31, 32 des Armbandes 30 nicht zurückgezogen oder gelöst werden, solange der Deckel 3 geschlossen ist. Ein Lösen ist erst möglich, wenn der Deckel wie oben beschrieben, mit dem Werkzeug 20 entfernt wird. Bei einem offenen Deckel 3 können die Enden 31, 32 des Armbandes 30 von den Dornen 9, 10 gehoben werden. Dazu kann mit einem Finger durch die Öffnung 11 Druck auf die Enden 31, 32 ausgeübt werden.

**Bezugszeichenliste**

| | | | |
|---|---|---|---|
| 1 | Gehäuse | 20 | Werkzeug |
| 2 | Unterteil | 21 | Zinken |
| 3 | Deckel | 22 | Querstrebe oder Sicherungsbügel |
| 4 | Boden | 23 | Einschnitt |
| 5 | Rand | 24 | Quersteg |
| 6 | Schlitz | 30 | Armband |
| 7 | Schlitz | 31 | Armbandende |
| 8 | Vertiefung | 32 | Armbandende |
| 9 | Dorn | 33 | Loch |
| 10 | Dorn | 34 | Loch |
| 11 | Öffnung | 35 | Mulde |
| 13 | Nase | 36 | Mulde |
| 14 | Nase | | |
| 15 | Aussparung | | |
| 16 | Aussparung | | |
| 17 | Federhaken | | |
| 18 | Sicherungsnase | | |

## Patentansprüche

1. Zur Aufnahme einer elektronischen und/oder mechanischen Einrichtung vorgesehenes Gehäuse (1) mit einem Armband (30) zum Befestigen des Gehäuses (1), insbesondere am Handgelenk oder Fußgelenk einer Person, und mit einer gesicherten Löse-Einrichtung zum Lösen des Armbandes (30) vom Gehäuse (1),
**dadurch gekennzeichnet,**
**dass** das Gehäuse (1) ein Unterteil (2) und einen über dem Unterteil (2) liegenden Deckel (3) aufweist,
**dass** das Armband (30) ein einteiliges Armband ist und zwei Enden (31, 32) aufweist, wobei mindestens eines der Enden (31, 32) auf der zum Deckel (3) gerichteten Innenseite des Unterteils (2) innerhalb der Erstreckungsabmessung des Deckels (3) lösbar befestigt ist,
**dass** der Deckel (3) mittels der gesicherten Löse-Einrichtung lösbar mit dem Unterteil (2) verbunden ist, und
**dass** die gesicherte Löse-Einrichtung so eingerichtet ist, dass sie mit einem zur gesicherten Löse-Einrichtung komplementären Löse-Werkzeug (20) lösbar ist.

2. Gehäuse nach Anspruch 1, **dadurch gekennzeichnet, dass**
das Armband (30) undehnbar ist, und/oder das Armband unter Aufbringung einer von einem Menschen aufbringbaren Kraft sich nicht dehnen lässt, und/oder
wobei das Armband eine Gleichmaßdehnung A_{g} aufweist wobei gilt A_{g}=Lₚₘ/L₀-1, mit Lₚₘ als plastischer Längenänderung und Lo als Anfangsmesslänge, und wobei gilt A_{g} = 0 oder 0 ≤ A_{g} ≤ 0,05 oder ≤ 0,01.

3. Gehäuse nach Anspruch 1 oder 2, **dadurch gekennzeichnet, dass** beide Enden (31, 32) des Armbands (30) auf der zum Deckel (3) gerichteten Innenseite des Unterteils (2) innerhalb der Erstreckungsabmessung des Deckels (3) lösbar befestigt sind.

4. Gehäuse nach einem der vorhergehenden Ansprüche, **dadurch gekennzeichnet, dass** sich auf dem zum Deckel (3) gerichteten Innenseite des Unterteils (2) mindestens ein Dorn (9, 10) befindet und dass das mindestens eine Ende (31, 32) des Armbandes (30) ein oder mehrere den Dorn aufnehmende Löcher (33, 34) aufweist.

5. Gehäuse nach Anspruch 4, **dadurch gekennzeichnet, dass** der mindestens eine Dorn (9, 10) bis zur Unterseite des Deckels (3) reicht, wenn dieser am Unterteil (2) befestigt ist.

6. Gehäuse nach Anspruch 5, **dadurch gekennzeichnet, dass** in der Unterseite des Deckels (3) je eine Mulde (35, 36) für jeden Dorn (9, 10) ausgebildet ist, in die dieser (9, 10) bei einem geschlossenen Deckel (3) hineinragt.

7. Gehäuse nach Anspruch 5 oder 6, **dadurch gekennzeichnet, dass** das Unterteil (2) einen Boden (4) mit einem zum Deckel (3) hin hochgezogenen Rand (5) aufweist, wobei sich im Rand (5) mindestens ein Schlitz (6, 7) befindet, durch den das mindestens eine Ende (31, 32) des Armbandes (30) eingeführt ist,
dass der Dorn (9, 10) vor dem Schlitz (6, 7) liegt, und
dass die gesicherte Löse-Einrichtung so hergerichtet ist, dass der Deckel (3) am Rand (5) lösbar befestigt ist.

8. Gehäuse nach einem der vorhergehenden Ansprüche, **dadurch gekennzeichnet, dass** die gesicherte Löse-Einrichtung als eine Schnappverbindung ausgeführt ist, so dass der Deckel (3) damit mit dem Unterteil (2) verbindbar ist, wobei die Schnappverbindung mindestens zwei voneinander beabstandete Federhaken (17) aufweist, deren Enden bei einem mit dem Unterteil (2) verbundenen Deckel (3) freiliegen, so dass das Werkzeug (20) an den freiliegenden Enden ansetzbar ist.

9. Löse-Werkzeug (20) zur Betätigung einer Löse-Einrichtung zur Öffnung des Gehäuses (1) nach einem der vorstehenden Ansprüche, umfassend eine Gabel mit mindestens zwei Zinken (21), deren Enden zum Betätigen der Einrichtung zum Lösen des Armbandes (30) zum Entfernen des Gehäuses (1) eingerichtet sind.

10. Löse-Werkzeug (20) nach dem vorstehenden Anspruch, **dadurch gekennzeichnet, dass** die Enden der Zinken (21) paarweise den gleichen Abstand wie jeweils zwei der Federhaken (17) am Gehäuse (1) haben.

11. Zur Aufnahme einer elektronischen und/oder mechanischen Einrichtung vorgesehenes Gehäuse (1), **dadurch gekennzeichnet,**
• **dass** das Gehäuse (1) ein Unterteil (2), einen über dem Unterteil liegenden Deckel (3) und eine gesicherte Löse-Einrichtung zum Öffnen des Gehäuses aufweist,
• **dass** der Deckel (3) mittels der Löse-Einrichtung lösbar mit dem Unterteil (2) verbunden ist, wobei die gesicherte Löse-Einrichtung so eingerichtet ist, dass sie mit einem Löse-Werkzeug (20) lösbar ist,
• **dass** sich auf der zum Deckel (3) gerichteten Innenseite des Unterteils (2) mindestens ein Dorn (9, 10) befindet,
• **dass** das Unterteil (2) einen Boden (4) mit einem zum Deckel hin hochgezogenen Rand (5) aufweist, wobei sich im Rand (5) mindestens ein Schlitz (6, 7) befindet, durch den das Ende eines Armbandes einführbar ist,
• **dass** der Dorn (6, 7) vor dem Schlitz (6, 7) liegt und
• **dass** die Löse-Einrichtung so hergerichtet ist, dass der Deckel (3) am Rand (5) lösbar befestigt ist.
